# FASCICULE DE BREVET EUROPEEN

(11) **EP 0 507 944 B1**
(45) Date de publication et mention de la délivrance du brevet: **28.09.1994**
(21) Numéro de dépôt: 92909593.3
(22) Date de dépôt: 22.10.1991
(51) Int. Cl.: A61K 9/22, A61K 9/20, A61L 27/00

(54) **MATERIAU IMPLANTABLE BIODEGRADABLE ET PROCEDE DE FABRICATION**
BIOABSORBIERBARES IMPLANTIERBARES MATERIAL UND VERFAHREN ZUR HERSTELLUNG
IMPLANTABLE BIODEGRADABLE MATERIAL AND METHOD FOR PRODUCING SAME

(30) Priorité: 26.10.1990 FR 9013424
(43) Date de publication de la demande: 14.10.1992
(73) Titulaire: CENTRE NATIONAL DE LA RECHERCHE SCIENTIFIQUE (CNRS), 75016 Paris (FR)
(72) Inventeur: LEBUGLE, Albert, F-31650 Saint-Orens (FR); JULIA, Anne, F-31380 Montastruc-la-Conseillère (FR); RODRIGUEZ, Fernand, F-31320 Castanet (FR); BONNEVIALLE, Paul, F-31100 Toulouse (FR)
(74) Mandataire: Barre, Philippe
(86) Numéro de dépôt international: FR9100837
(87) Numéro de publication internationale: WO9207554

(56) Documents cités:
- FR-A- 2 570 606

## Description

L'invention concerne un nouveau matériau implantable dans un tissu vivant, en particulier tissu osseux. Elle vise un matériau ayant une vitesse de biodégradation adaptée à la régénération du tissu et susceptible le cas échéant d'être associé avec une substance active pour produire un effet thérapeutique prolongé.

On connaît des matériaux ou matrices d'implantation dans les tissus vivants, notamment tissus osseux, qui permettent de combler un "defect" du tissu et le cas échéant de libérer une substance active. On peut actuellement classer ces matrices d'implantation en quatre catégories.

En premier lieu, il existe des matrices non biodégradables telles que certains polymères (par exemple polyméthylméthacrylate PMMA) qui sont utilisées pour libérer localement des substances actives et éviter ainsi leur administration par voie générale. Ces matrices ont le défaut de nécessiter une nouvelle intervention chirurgicale pour les extraire après libération de la substance active (brevet FR 74.13342). De plus une très faible fraction de la substance active de départ est libérée (6 % environ).

D'autres matrices plus anciennes telles que matrices à base de sulfate de calcium, sont biodégradables, mais leur vitesse de dégradation est fixe et ne peut être adaptée à la vitesse de régénération du tissu considéré ; leur dégradation étant généralement trop rapide pour les tissus osseux, elles entraînent fréquemment l'apparition de "defects" dans ces tissus. Elles présentent également l'inconvénient de ne pas favoriser la régénération du tissu.

Un autre type de matrice d'implantation est constitué par les matrices polylactiques-glycoliques PLGA qui ont l'avantage, grâce à un dosage relatif des deux composants, de permettre une certaine modulation de leur vitesse de dégradation afin d'adapter celle-ci à la vitesse de régénération du tissu considéré. Toutefois, ces matrices n'ont aucun effet favorable sur la régénération des tissus et, défaut plus grave elles entraînent des réactions inflammatoires, dues à la dépolymérisation en cours de dégradation (formation d'acides).

D'autres polymères ont été utilisés comme obturateur de puits fémoral ou de fond de cotyle afin d'éviter une fuite du ciment osseux disposé dans le canal médullaire lors d'une arthroplastie (brevet FR-A-2.570.606). Toutefois, les matériaux prévus ne sont pas destinés à remplacer le tissu osseux lui-même et font simplement office d'obturateur. De plus, la durée de biodégradabilité est très courte puisqu'ils doivent rester en place uniquement jusqu'à durcissement du ciment. Comme pour les matrices PLGA ci-dessus évoquées, ce type de matériau ne permet aucune modulation de la vitesse de dégradation sur de longues durées et ne présente aucun effet favorable sur la régénération des tissus.

La dernière catégorie de matrices d'implantation est constituée par des matrices à base de phosphate tricalcique ou apatitique dont la cohésion est assurée soit par frittage (céramique poreuse telle que visée dans le brevet DE 2.807.132), soit par un liant tel que collagène ou élastine (brevet DE 3.206.726). Ces matrices favorisent la régénération osseuse mais leur biodégradabilité est très lente et n'est pas modulable. Dans ces conditions, elles font obstacle à une reconstitution normale des tissus malgré leur effet positif sur l'ostéogénèse. De plus, lorsqu'elles sont associées a une substance active de type soluble, par exemple sulfate de gentamicine, elles conduisent à une libération beaucoup trop rapide de la substance, représentant un inconvénient majeur dans le cas de substances à toxicité élevée et/ou dans le cas d'un traitement nécessitant une couverture prolongée. Pour pallier ces défauts, l'on transforme parfois la substance active en un sel peu soluble (par exemple phosphate hespéridine cité dans le brevet DE 3.206.706) : grâce à un certain caractère hydrophobe conféré au produit, on retarde ainsi sa libération sur le site mais celle-ci reste très faible au début de l'implantation et ne devient efficace qu'après un laps de temps notable, ce qui représente un inconvénient grave dans le traitement d'une affection.

Par ailleurs, le brevet EP 0.147.021 décrit une composition médicamenteuse liquide (suspension aqueuse) qui peut être amenée dans la cavité d'un os au moyen d'un tube de drainage en vue de traiter localement une affection (ostéomyélite) par libération de la substance active. Toutefois, il ne s'agit pas d'un matériau d'implantation capable de remplacer temporairement le tissu mais d'une simple solution n'ayant aucune propriété mécanique qui est uniquement destinée à libérer localement la substance active, puis à s'éliminer. De plus, la libération de ladite substance active est immédiate et n'a aucun effet prolongé dans le temps.

La présente invention se propose de fournir un nouveau matériau implantable du type à base de phosphate de calcium, ayant une parfaite biocompatibilité et susceptible de favoriser l'ostéogénèse sans aucune réaction préjudiciable telle que réaction inflammatoire ; l'objectif essentiel de l'invention est de fournir un matériau implantable qui présente des propriétés mécaniques lui permettant de se substituer temporairement au tissu défectueux et qui bénéficie d'une vitesse de biodégradation ajustée à la vitesse de régénération dudit tissu.

Un autre objectif de l'invention est d'associer le matériau sus-visé à une substance active dotée de groupements appropriés pour assurer une libération progressive de ladite substance avec un effet thérapeutique prolongé dans le temps.

Un autre objectif est de fournir un matériau d'implantation facile à fabriquer à froid par compaction à partir de ces divers constituants.

A cet effet, le procédé visé par l'invention pour fabriquer le matériau implantable sus-visé consiste à mélanger un phosphate de calcium et un oside ou polyoside biodégradable, et se caractérise en ce que :
. l'on choisit un phosphate de calcium de structure apatitique ou triclinique contenant à la fois des groupements HPO₄ et des groupements PO₄,
. l'on mélange une proportion d'oside ou polyoside de valeur fonction croissante de la vitesse de dégradation désirée pour le matériau.
Le phosphate de calcium utilisé peut contenir différents substituants cationiques du calcium (magnésium, sodium) ou substituants anioniques des ions phosphates (carbonates) ou substituants des ions hydroxyles (chlore) ; il peut également être non substitué et répondre à la formule :

Ca_{8+y}(HPO₄)_{2+x-y}(PO₄)_{4-x+y}OH_{x+y}

avec 0 ≦ y ≦ 2 et 0 ≦ x < 1.

Le phosphate de calcium du type précité et l'oside ou polyoside sont de préférence mélangés sous forme de poudre puis compactés pour leur donner une forme prédéterminée. Les implants ainsi fabriqués peuvent être disposés dans le tissu, notamment tissu osseux après réalisation d'un logement adapté à l'emplacement du "defect" ou bien immobilisés dans une cavité existante du tissu à l'aide d'un ciment biodégradable tel que sulfate de calcium ou ciment à base de phosphate.

Les expérimentations ont permis de constater que la dégradation du matériau implantable conforme à l'invention variait entre une durée de l'ordre de quelques jours (3-4 jours) et une durée beaucoup plus longue de l'ordre de quelques mois (10-12 mois) en fonction de la proportion d'oside ou polyoside mélangé au phosphate. Différents osides ou polyosides peuvent être utilisés, les durées de dégradation augmentant avec la masse moléculaire de ce composé ; ainsi l'on peut moduler la vitesse de biodégradation par un choix de l'oside ou polyoside et par un ajustement de sa teneur par rapport au phosphate. En particulier l'on peut utiliser comme polyoside, du dextran, la proportion de celui-ci étant modulée entre 1 % et 30 % en fonction de la vitesse de dégradation désirée ; cette dernière est une fonction croissante de la proportion de dextran, l'on mélangera donc au phosphate des quantités de dextran d'autant plus grandes que l'on désirera des durées de dégradation plus courtes.

Par ailleurs, le matériau implantable conforme à l'invention peut être associé à une substance active apte à produire un effet thérapeutique désiré. Selon une caractéristique de l'invention, l'on choisit une substance active possédant des groupements amines permettant sa fixation sur le phosphate de calcium pourvu des groupements HPO₄ et PO₄.

Les trois constituants du matériau de l'invention coopèrent alors pour engendrer une libération progressive de la substance active. On a pu mettre en évidence que la fixation de la substance active sur le phosphate est liée à la présence simultanément des groupements HPO₄ et PO₄, probablement par adsorption grâce aux groupements amines de ladite substance, cette fixation étant de nature à conduire ensuite à une libération progressive de celle-ci sur le site d'implantation. De façon surprenante, les essais ont permis de constater que la présence des osides ou polyosides avaient la propriété de régulariser cette libération, permettant d'atteindre immédiatement des taux efficaces et de prolonger celle-ci sur plusieurs semaines avec un taux sensiblement constant. (On pourrait s'attendre à un effet opposé puisque les osides ou polyosides sont des composés très solubles).

Il convient de souligner, en outre, que les osides ou polyosides jouent un rôle de plastifiant lors de la compaction du matériau, améliorant considérablement sa cohésion ; cet effet semble provenir de ce que la présence de ce composé entraîne une augmentation notable de la taille des grains de phosphate, ce qui accroît la cohésion ; ce phénomène d'augmentation de la taille des grains permet d'expliquer au moins en partie la diminution et la régularisation de la vitesse de libération de la substance active : cette substance fixée grâce aux groupements HPO₄ et PO₄ du phosphate se détache plus difficilement à l'intérieur de grains de grosse taille ; en outre la diminution de la surface spécifique des grains, lorsque leur taille augmente, est un facteur de réduction de la cinétique de dissolution aux joints de grains.

En résumé, dans l'invention, l'effet retard et ses caractéristiques (vitesse de libération sensiblement constante et effet prolongé dans le temps) sont obtenus par une fixation de la substance active sur la matrice ; cette substance qui se présente de préférence sous forme soluble, est libérée par rupture de ces liaisons ou interactions, avec une influence positive de l'oside ou du polyoside sur la force de celles-ci. (Au contraire, dans le brevet DE 3.206.726 déjà évoqué, la matrice est inerte vis-à-vis de la substance active et l'effet retard du médicament est dû à la forme insoluble donnée à la substance active).

Le phosphate de calcium, la substance active et l'oside ou polyoside sont de préférence mélangés sous forme de poudre et compactés ; ce compactage facile confère au matériau une bonne cohésion avec les pressions de compactage habituelles : le matériau peut ainsi être fabriqué à froid sans altération de la substance active (généralement sensible à la chaleur).

Selon une forme préférée du matériau, on choisit comme phosphate, un phosphate octocalcique apatitique de formule : Ca₈(HPO₄)₂₊ₓ(PO₄)₄₋ₓ(OH)ₓ, avec 0 < x < 1. Ce phosphate est celui qui possède le nombre de groupements HPO₄ le plus élevé par unité formulaire ; il fournit dans le cadre de l'invention les meilleures performances (meilleure fixation de la substance active par unité de masse de matériau, propriétés galéniques améliorées). De bons résultats sont obtenus en choisissant pour le paramètre x une valeur de l'ordre de 0,5 , (à ∓ 20 % près), la formule du matériau utilisée étant alors proche de la suivante :

Ca₈(HPO₄)_{2,5}(PO₄)_{3,5}(OH)_{0,5}

La fabrication de tels phosphates est connue en elle-même ; des exemples sont fournis plus loin avec les références de publications décrivant des méthodes de fabrication.

La substance active mélangée se présente avantageusement sous la forme d'un sel soluble, en particulier dans le cas de la nétilmicine et/ou de la gentamicine, sous la forme d'un sulfate (qui en est la forme la plus soluble) ; ces substances sont d'un usage très fréquent dans les affections osseuses en raison de leur large spectre d'efficacité.

A titre d'exemple non limitatif, le matériau conforme à l'invention peut être constitué par un mélange de phosphate octocalcique apatitique de formule précitée (paramètre x voisin de 0,5), de dextran et de nétilmicine et/ou gentamicine sous la forme sulfate, dans les proportions suivantes :
. la proportion pondérale de dextran est comprise entre 1 % et 30 % par rapport au mélange (en fonction de la vitesse de biodégradation désirée),
. la proportion pondérale de substance active est comprise entre 0,1 % et 30 % par rapport au mélange (en fonction de l'activité de la substance et des effets désirés).

La description qui suit présente des exemples de matériaux d'implantation conformes à l'invention, et illustre leurs propriétés galéniques, pharmacocinétiques et physiologiques, ainsi que l'évolution de celles-ci en fonction de la composition.

### EXEMPLE 1

### Préparation du phospate OCPa

100 g de phosphate octocalcique de structure apatitique de formule Ca₈(HPO₄)_{2,5}(PO₄)_{3,5}(OH)_{0,5} sont préparés par précipitation dans un milieu obtenu en ajoutant les mêmes volumes d'éthanol et de solution aqueuse, à partir d'une solution (A) de sel de calcium et d'une solution ammoniacale (B) contenant des ions orthophosphate. Le nombre d'ions calcium contenu dans la solution (A) est égal au nombre d'ions phosphate dans la solution (B). La solution (A) est versée rapidement à 37° C, sous agitation, dans la solution (B). Après formation, le précipité est filtré sur Buchner, puis lavé avec une solution basique, et séché à l'étuve à 80° C. On obtient une poudre qui présente une répartition granulométrique quasi-homogène comprise entre 0,5 et 0,08 mm, un temps d'écoulement (analyse rhéologique) de 4 s (normes AFNOR). L'étude de la compression de cette poudre réalisée à l'aide d'une machine à comprimer alternative "Korsch type EKO", équipée de jauges de contraintes pour pouvoir mesurer les paramètres de compression, révèle un travail d'agrégation de 50 %, une transmission de 84 %.

### Fabrication d'un matériau sans effet thérapeutique

Du dextran (Fournisseur "SIGMA, qualité "Clinical Grade"") a été ajouté en proportion variable à du phosphate octocalcique ci-dessus préparé ; la teneur en dextran dans le mélange a été amenée à varier entre 1 % et 30 % (un test comparatif est réalisé en l'absence de dextran). Les deux composés sont mélangés intimement et comprimés sous une pression de 2 000 DaN/cm² environ, à la température ordinaire. Les comprimés obtenus, de diamètre 6 mm et d'épaisseur 2 mm, ont une dureté Stockes de 15 et une friabilité nulle en présence de dextran, tandis qu'en l'absence de ce dernier (OCPa pur), la dureté n'est que de 10, et la friabilité de 1 %.

### EXEMPLE 2

80 g de phosphate octocalcique tel que préparé à l'exemple 1 sont mélangés intimement à 20 g de glucose et comprimés dans les conditions précédentes. Les comprimés obtenus présentent une dureté Stockes de 14 et une friabilité nulle.

### EXEMPLE 3

### Fabrication d'un matériau avec effet thérapeutique

90 g de phosphate octocalcique tel que préparé à l'exemple 1, 5 g de nétilmicine sulfate et 5 g de dextran sont mélangés intimement et comprimés sous une pression de 2 000 DaN/cm² environ, à température ordinaire. Les comprimés obtenus, de diamètre 6 mm et d'épaisseur 2 mm, ont une dureté Stockes de 15 et une friabilité nulle;

### EXEMPLE 4

85 g de phosphate octocalcique préparé comme dans l'exemple 1, 5 g de nétilmicine sulfate et 10 g de dextran sont mélangés intimement et comprimés sous une pression de 2 000 DaN/cm², à température ordinaire sous forme de pastilles de 6 mm de diamètre et 2 mm d'épaisseur. La dureté Stockes mesurée est de 18 et la friabilité nulle.

### EXEMPLE 5

80 g de phosphate octocalcique préparé comme dans l'exemple 1, 5 g de nétilmicine sulfate et 15 g de dextran sont mélangés intimement et comprimés sous une pression de 2 000 DaN/cm², à température ordinaire sous forme de pastilles de 6 mm de diamètre et 2 mm d'épaisseur. La dureté Stockes mesurée est de 18 et la friabilité nulle.

### EXEMPLE 6

75 g de phosphate octocalcique préparé comme dans l'exemple 1, 5 g de nétilmicine sulfate et 20 g de dextran sont mélangés intimement et comprimés sous une pression de 2 000 DaN/cm², à température ordinaire sous forme de pastilles de 6 mm de diamètre et 2 mm d'épaisseur. La dureté Stockes mesurée est de 17 et la friabilité nulle.

### EXEMPLE 7

Dans cet exemple, la teneur de nétilmicine a été amenée à varier de même que la teneur en phosphate de façon que la somme des deux demeure constante et égale à 95 %, la teneur en dextran étant constante et égale à 5 %.

Le phosphate octocalcique a été fabriqué comme à l'exemple 1. Quatre médicaments aux teneurs suivantes ont été successivement obtenus :
. 85 % de phosphate octocalcique, 10 % de nétilmicine sulfate et 5 % de dextran,
. respectivement 80 %, 15 % et 5 %,
. respectivement 75 %, 20 % et 5 %,
. respectivement 70 %, 25 % et 5 %.

La compaction a été similaire a celle des exemples précédents et a conduit à une dureté Stockes sensiblement la même et égale à 17 et à une friabilité nulle.

### EXEMPLE 8

90 g de phosphate tricalcique de structure apatitique et de formule Ca₉(HPO₄) (PO₄)₅OH ont été préparés selon un protocole analogue à l'exemple 1, à la différence que le rapport du nombre d'ions calcium dans la solution (A), sur le nombre d'ions phosphore dans la solution (B) n'est plus égal à 1 mais à 1,6 ; 5 g de nétilmicine sulfate et 5 g de dextran ont été mélangés intimement avec ces 85 g de phosphate tricalcique et comprimés sous une pression de 2 000 DaN/cm², à température ordinaire. La dureté Stockes mesurée est de 13 et la friabilité de 0,5 % en poids.

### EXEMPLE 9

Du phosphate octocalcique de structure triclinique, et de formule Ca₈(HPO₄)₂(PO₄)₄, a été préparé selon la méthode de BROWN, qui consiste en une hyrolyse lente de la brushite Ca(HPO₄)2H₂O dans une solution 0,5 M d'acétate de sodium à 40° C (W. BROWN et al., J. Am. Chem. Soc. 79 -p. 5318-9. 1957), 85 g de ce phosphate, 5 g de nétilmicine sulfate et 10 g de dextran ont été mélangés intimement et comprimés dans les conditions précédentes. Les comprimés ont une dureté de 15 et une friabilité nulle.

### EXEMPLE 10

90 g de phosphate octocalcique préparé comme dans l'exemple 1, 5 g de gentamicine sulfate et 5 g de dextran ont été mélangés intimement et comprimés comme précédemment. Les comprimés ont une dureté de 15 et une friabilité nulle.

### EXEMPLE 11

85 g de phosphate octocalcique de structure apatitique préparé comme à l'exemple 1, 5 g de nétilmicine sulfate et 10 g de glucose ont été mélangés intimement et comprimés. Les comprimés ont une dureté de 16 et une friabilité de 0,5 %.

### EXEMPLE 12

L'influence de la teneur en dextran sur la dégradation des matériaux réalisés dans l'exemple 1 a été étudiée par implantation après forage d'une cavité chez le chien, dans l'ulna, en région métaphysaire proximale, ainsi que dans le radius en zone métaphysaire distale. On constate par radiographie et étude anatomopathologique qu'après deux mois, les matériaux implantés ne sont dégradés qu'en périphérie lorsque la teneur en dextran est nulle (test), et que la dégradation augmente avec cette teneur. Lorsque cette dernière est comprise entre 20 et 30 %, l'implant a disparu et a été recolonisé par de l'os spongieux. A aucun moment au cours des deux mois, les radiographies de contrôle n'ont pu mettre en évidence de "defect" entre l'implant et l'os.

### EXEMPLE 13

L'influence de la nature de l'oside ou polyoside sur la dégradation des matériaux réalisés dans les exemples 1 et 2 a été étudiée comme dans l'exemple 12, par implantation chez le chien. On constate qu'à une teneur de 20 % de glucose, l'implant a été totalement dégradé au bout de deux mois et qu'il subsiste un "defect" osseux, tandis que dans le cas du dextran, l'implant est dégradé mais a été remplacé par de l'os. A la teneur étudiée (20 %), les matériaux réalisés avec du glucose sont trop rapidement dégradés pour les tissus osseux concernés (ulna et radius) et ne permettent pas une repousse osseuse, tandis que cette dernière a lieu dans le cas du dextran où la vitesse de dégradation est adaptée à celle de la régénération osseuse.

### EXEMPLE 14

L'influence de la teneur en dextran sur la cinétique de libération de la nétilmicine a été étudiée in vitro dans un appareil à dissolution. Cette étude a été conduite à l'aide des comprimés réalisés dans les exemples 3 à 6. Le protocole de libération a été le suivant : pour chaque médicament, deux comprimés de 100 mg ont été placés en dissolution dans la cuve de l'appareil contenant 500 ml d 'eau distillée et thermostatée à 37° C sous une agitation continue (60 tr/mn). Des prélèvements ont été effectués périodiquement. La nétilmicine a été dosée par immuno-enzymologie.

On constate que paradoxalement, la quantité d'antibiotique libéré après 50 h de dissolution conduite à 37° C, diminue lorsque la proportion de dextran augmente, bien que de dernier soit hydrophyle. Ce caractère hydrophyle exerce cependant une influence au début de la cinétique où il accroît avantageusement la libération de la nétilmicine permettant d'atteindre très rapidement des concentrations élevées, supérieures à la C.M.I. ("Concentration Minimale Inhibitrice").

### EXEMPLE 15

L'influence de la nature de l'antibiotique sur sa cinétique de libération a été étudiée in vitro dans un appareil à dissolution à l'aide de comprimés réalisés à l'exemple 3 et, dans un but de comparison, à l'aide de comprimés réalisés dans les mêmes conditions, mais en substituant à la nétilmicine, une substance active ne comportant pas de groupements amines, à savoir l'oxacilline. Cette étude révèle qu'une libération totale et très rapide (1 h) est observée dans le cas de l'oxacilline, tandis que cette libération n'est que de 30 %, au bout de 50 h, dans les mêmes conditions expérimentales avec la nétilmicine. La présence de groupements amines dans les aminosides exerce une influence déterminante sur les mécanismes de libération du principe actif.

### EXEMPLE 16

L'influence de la composition chimique du phosphate sur la cinétique de libération de l'aminoside a été étudiée in vitro dans un appareil de dissolution à l'aide de comprimés réalisés aux exemples 3 et 8, et pour comparaison, à l'aide de comprimés réalisés dans les mêmes conditions, mais en substituant au phosphate octocalcique ou tricalcique de structure apatitique, de l'hydroxyapatite qui ne possède pas de groupement HPO₄ (formule Ca₁₀(PO₄)₆(OH)₂). Ce dernier composé a été préparé selon la méthode de TROMBE qui consiste à verser lentement une solution de phosphate diammonique dans une solution d'acétate de calcium, le rapport du nombre d'ions calcium sur le nombre d'ions phosphore dans ces solutions étant égal à 1,66 (J.C. TROMBE, Ann. Chim. 8, 251, 1973). Cette étude met en évidence que les cinétiques de libération sont directement reliées à la teneur en ions hydrogénophosphate, HPO₄, dans les phosphates examinés. Ainsi, la libération de l'aminoside est quasi-immédiate dans le cas de l'hydroxyapatite (teneur en HPO₄ nulle), ralentie dans le cas du phosphate tricalcique (teneur en HPO₄ égale à 1) avec un taux de libération de 60 % au bout de 50 h, et extrêmement ralentie dans le cas du phosphate octocalcique de structure apatitique, avec un taux de 30 % après 50 h (teneur en HPO₄ proche de 2,5).

### EXEMPLE 17

L'influence de la nature de l'oside utilisé sur la cinétique de libération de la substance active a été étudiée in vitro dans les mêmes conditions que celles de l'exemple 15 au moyen de comprimés réalisés dans les exemples 4 et 11. Cette étude comparative a montré que les osides utilisés exercent un effet sensiblement comparable sur la cinétique de libération, avec toutefois une préférence pour le dextran qui conduit à une libération plus lente.

### EXEMPLE 18

L'influence de la formulation des comprimés sur leur comportement in vivo, a été étudiée en les implantant en site osseux chez l'animal, lapin et chien, et en examinant leur biocompatibilité et leur vitesse de dégradation. Cette étude a été conduite à l'aide de comprimés réalisés aux exemples 3 à 11 ; de plus, à titre comparatif, des médicaments ont été fabriqués dans les mêmes conditions avec des teneurs en dextran et nétilmicine soit trop faibles (teneur totale inférieure à 1 %), soit trop élevées (teneur totale supérieure à 35 %). Lorsque la teneur totale en dextran et nétilmicine est trop faible, l'implant bien que parfaitement toléré, n'est pas complètement dégradé après trois mois d'implantation. Dans cet exemple d'implantation en site osseux, lorsque la teneur totale en dextran et nétilmicine est trop élevée, l'implant a été dégradé trop rapidement, et il subsiste un "defect" osseux. Par contre, lorsque la teneur globale en dextran et de nétilmicine est comprise entre 4 % et 35 %, l'implant parfaitement biocompatible, a été dégradé en grande partie après trois mois, et recolonisé par de l'os spongieux ; les meilleurs résultats ont été obtenus avec les médicaments des exemples 3 à 6. La formulation des comprimés permet ainsi d'ajuster la vitesse de dégradation à l'objectif souhaité.

## Revendications

1. Procédé de fabrication d'un matériau implantable dans un tissu vivant, permettant d'obtenir un matériau ayant une vitesse de biodégradation ajustable en fonction de la vitesse de régénération du tissu concerné, le procédé consistant à mélanger un phosphate de calcium et un oside ou polyoside biodégradable, et étant caractérisé en ce que :
. l'on choisit un phosphate de calcium de structure apatitique ou triclinique contenant à la fois des groupements HPO₄ et des groupements PO₄,
. l'on mélange une proportion d'oside ou polyoside de valeur fonction croissante de la vitesse de dégradation désirée pour le matériau.

2. Procédé selon la revendication 1, caractérisé en ce que l'on choisit un phosphate de calcium de formule :
Ca_{8+y}(HPO₄)_{2+x-y}(PO₄)_{4-x+y}OH_{x+y}
avec 0 ≦ y ≦ 2 et 0 ≦ x < 1.

3. Procédé selon l'une des revendications 1 ou 2, caractérisé en ce que l'on mélange le phosphate de calcium et l'oside ou polyoside sous forme de poudre et en ce que l'on compacte le mélange.

4. Procédé selon l'une des revendications 1, 2 ou 3, en vue de fabriquer un matériau implantable ayant un effet thérapeutique prolongé, caractérisé en ce que l'on ajoute au mélange une substance active apte à produire l'effet thérapeutique désiré et possédant des groupements amines permettant sa fixation sur le phosphate de calcium pourvu des groupements HPO₄ et PO₄.

5. Procédé selon la revendication 4, caractérisé en ce que l'on mélange le phosphate de calcium, la substance active et l'oside ou polyoside sous forme de poudre et en ce que l'on compacte le mélange.

6. Matériau implantable dans un tissu vivant, fabriqué par mise en oeuvre du procédé conforme à l'une des revendications 1, 2 ou 3 en vue de posséder une vitesse de biodégradation adaptée au tissu, comprenant :
(a) un phosphate de calcium de structure apatitique ou triclinique contenant à la fois des groupements HPO₄ et des groupements PO₄,
(b) un oside ou un polyoside biodégradable.

7. Matériau selon la revendication 6, dans lequel le phosphate de calcium est un phosphate de formule :
Ca_{8+y}(HPO₄)_{2+x-y}(PO₄)_{4-x+y}OH_{x+y}
avec 0 ≦ y ≦ 2 et 0 ≦ x < 1

8. Matériau implantable dans un tissu vivant, présentant un effet thérapeutique prolongé, fabriqué par mise en oeuvre du procédé conforme à l'une des revendications 4 ou 5, comprenant :
(a) un phosphate de calcium de structure apatitique ou triclinique contenant à la fois des groupements HPO₄ et des groupements PO₄,
(b) un oside ou un polyoside biodégradable,
(c) au moins une substance active possédant des groupements amines et apte à produire l'effet thérapeutique désiré.

9. Matériau selon la revendication 8, dans lequel le phosphate de calcium est un phosphate de formule :
Ca_{8+y}(HPO₄)_{2+x-y}(PO₄)_{4-x+y}OH_{x+y}
avec 0 ≦ y ≦ 2 et 0 ≦ x < 1.

10. Matériau implantable selon l'une des revendications 6 à 9, dans lequel (a) le phosphate de calcium est constitué par un phosphate octocalcique apatitique de formule :
Ca₈(HPO₄)₂₊ₓ(PO₄)₄₋ₓ(OH)ₓ
avec 0 < x < 1.

11. Matériau implantable selon la revendication 10, dans lequel (a) le phosphate est le phosphate octocalcique apatitique dont le paramètre x est voisin de 0,5 (à ∓ 20 % près), de formule proche de :
Ca₈(HPO₄)_{2,5}(PO₄)_{3,5}(OH)_{0,5}

12. Matériau implantable selon l'une des revendications 6 à 11, dans lequel (b) le polyoside est constitué par du dextran.

13. Matériau implantable selon la revendication 12, dans lequel la proportion pondérale de dextran est comprise entre 1 % et 30 % par rapport au mélange.

14. Matériau implantable selon la revendication 8, dans lequel (c) la substance active se présente sous la forme d'un sel soluble dans l'eau.

15. Matériau implantable selon la revendication 14, dans lequel la substance active est la nétilmicine et /ou la gentamicine sous la forme d'un sulfate.

16. Matériau implantable selon la revendication 8, comprenant :
. une proportion pondérale de dextran comprise entre 1 % et 30 % par rapport au mélange,
. une proportion pondérale de substance active comprise entre 0,1 % et 30 % par rapport au mélange.

## Claims

1. Process for manufacturing a material capable of being implanted in a living tissue and making it possible to produce a material having a rate of biodegradation which can be adjusted as a function of the rate of regeneration of the tissue concerned, said process consisting in mixing a calcium phosphate with a biodegradable oside or polyoside, and characterised in that:
. a calcium phosphate of apatitic or triclinic structure is selected which contains both HPO₄ and PO₄ groups.
. a proportion of oside or polyoside is mixed which is larger, the higher the desired rate of degradation of the material.

2. Process according to claim 1, characterised in that a calcium phosphate is selected having the formula:
Ca_{8+y}(HPO₄)_{2+x-y}(PO₄)_{4-x+y}OH_{x+y}
where 0 ≦ y ≦ 2 and 0 ≦ x < 1.

3. Process according to one of Claims 1 or 2, characterised in that the calcium phosphate and the oside or polyoside are mixed in the form of powder and in that the mixture is compacted.

4. Process according to one of Claims 1, 2 or 3, with a view to manufacturing a material capable of implantation and having a prolonged therapeutic effect, characterised in that an active substance capable of producing the desired therapeutic effect is added to the mixture, said active substance comprising amine groups enabling its attachment to the calcium phosphate endowed with HPO₄ and PO₄ groups.

5. Process according to Claim 4, characterised in that the calcium phosphate, the active substance and the oside or polyoside are mixed in the form of powder and in that the mixture is compacted.

6. Material capable of implantation into a living tissue, manufactured by implementing the process according to one of Claims 1, 2 or 3, with a view to achieving a rate of biodegradation adapted to the tissue, comprising:
(a) a calcium phosphate of apatitic or triclinic structure containing both HPO₄ groups and PO₄ groups.
(b) a biodegradable oside or polyoside.

7. Material according to Claim 6, in which the calcium phosphate is a phosphate with the formula:
Ca_{8+y}(HPO₄)_{2+x-y}(PO₄)_{4-x+y}OH_{x+y}
where 0 ≦ y ≦ 2 and 0 ≦ x < 1.

8. Material capable of implantation into a living tissue exhibiting a prolonged therapeutic effect and manufactured by implementing the process according to one of Claims 4 or 5, comprising:
(a) a calcium phosphate of apatitic or triclinic structure containing both HPO₄ groups and PO₄ groups,
(b) a biodegradable oside or polyoside,
(c) at least one active substance comprising amine groups and capable of producing the desired therapeutic effect.

9. Material according to Claim 8 in which the calcium phosphate is a phosphate with the formula:
Ca_{8+y}(HPO₄)_{2+x-y}(PO₄)_{4-x+y}OH_{x+y}
where 0 ≦ y ≦ 2 and 0 ≦ x < 1.

10. Material capable of implantation according to one of Claims 6 to 9, in which (a) the calcium phosphate is constituted by an apatitic octacalcic phosphate with the formula:
Ca₈(HPO₄)₂₊ₓ(PO₄)₄₋ₓ(OH)ₓ
where 0 < x < 1.

11. Material capable of implantation according to Claim 10, in which (a) the phosphate is the apatitic octacalcic phosphate, the parameter x of which is close to 0.5 (within about ± 20%), the formula of said octacalcic phosphate being approximately:
Ca₈(HPO₄)_{2.5}(PO₄)_{3.5}(OH)_{0.5}

12. Material capable of implantation according to one of Claims 6 to 11, in which (b) the polyoside is constituted by dextran.

13. Material capable of implantation according to Claim 12, in which the proportion by weight of dextran amounts to between 1% and 30% in relation to the mixture.

14. Material capable of implantation according to Claim 8, in which (c) the active substance is present in the form of a water-soluble salt.

15. Material capable of implantation according to Claim 14, in which the active substance is netilmycine and/or gentamycine in the form of a sulphate.

16. Material capable of implantation according to Claim 8, comprising :
. a proportion by weight of dextran between 1% and 30% in relation to the mixture,
. a proportion by weight of active substance between 0.1% and 30% in relation to the mixture.

## Patentansprüche

1. Verfahren zur Herstellung eines in ein lebendes Gewebe implantierbaren Stoffes, das die Erzielung eines Stoffes mit einer in Abhängigkeit von der Wiederherstellungsgeschwindigkeit des betreffenden Gewebes einstellbaren Geschwindigkeit biologischen Abbaus gestattet, wobei das besagte Verfahren darin besteht, daß ein Calciumphosphat mit einem biologisch abbaufähigen Osid oder Polyosid gemischt wird, und zwar ist das besagte Verfahren dadurch gekennzeichnet,
. daß man ein Calciumphosphat mit apatitartiger oder trikliner Struktur wählt, das gleichzeitig HPO₄- und PO₄-Gruppen enthält,
. daß der Anteil des beigemischten Osids oder Polyosids um so höher ist, je höher die bei dem Stoff angestrebte Abbaugeschwindigkeit ist.

2. Verfahren nach Anspruch 1, dadurch gekennzeichnet, daß man ein Calciumphosphat mit der Formel
Ca_{8+y}(HPO₄)_{2+x-y}(PO₄)_{4-x+y}OH_{x+y}
wählt, wobei 0 ≦ y ≦ 2 und 0 ≦ x < 1.

3. Verfahren nach einem der Ansprüche 1 oder 2, dadurch gekennzeichnet, daß man das Calciumphosphat und das Osid oder Polyosid in Pulverform mischt und das Gemisch verdichtet.

4. Verfahren nach einem der Ansprüche 1, 2 oder 3 für die Herstellung eines implantierbaren Stoffes mit anhaltender therapeutischer Wirkung, dadurch gekennzeichnet, daß man dem Gemisch einen Wirkstoff zusetzt, der geeignet ist, die gewünschte therapeutische Wirkung zu erzeugen, und Amingruppen umfaßt, die seine Anlagerung an das mit HPO₄- und PO₄-Gruppen versehene Calciumphosphat ermöglichen.

5. Verfahren nach Anspruch 4, dadurch gekennzeichnet, daß man das Calciumphosphat, den Wirkstoff und das Osid oder Polyosid in Pulverform mischt und daß man das Gemisch verdichtet.

6. In ein lebendes Gewebe implantierbarer Stoff, der durch Durchführung des Verfahrens nach einem der Ansprüche 1, 2 oder 3 hergestellt wird, um eine dem Gewebe entsprechende Geschwindigkeit biologischen Abbaus zu erzielen, umfassend:
(a) ein Calciumphosphat mit apatitartiger oder trikliner Struktur, das gleichzeitig HPO₄-Gruppen und PO₄-Gruppen umfaßt,
(b) ein biologisch abbaufähiges Osid oder Polyosid.

7. Stoff nach Anspruch 6, bei dem das Calciumphosphat ein Phosphat mit der Formel
Ca_{8+y}(HPO₄)_{2+x-y}(PO₄)_{4-x+y}OH_{x+y}
ist, wobei 0 ≦ y ≦ 2 und 0 ≦ x < 1.

8. In ein lebendes Gewebe implantierbarer Stoff mit anhaltender therapeutischer Wirkung, hergestellt durch Durchführung des Verfahrens nach einem der Ansprüche 4 oder 5, umfassend:
(a) ein Calciumphosphat mit apatitartiger oder trikliner Struktur, das gleichzeitig HPO₄-Gruppen und PO₄-Gruppen umfaßt.
(b) ein biologisch abbaufähiges Osid oder Polyosid.
(c) mindestens einen Amingruppen enthaltenden Wirkstoff, der geeignet ist, die gewünschte therapeutische Wirkung zu erzeugen.

9. Stoff nach Anspruch 8, bei dem das Calciumphosphat ein Phosphat der Formel
Ca_{8+y}(HPO₄)_{2+x-y}(PO₄)_{4-x+y}OH_{x+y}
ist, wobei 0 ≦ y ≦ 2 und 0 ≦ x < 1.

10. Implantierbarer Stoff nach einem der Ansprüche 6 bis 9, bei dem (a) das Calciumphosphat ein apatitartiges Octacalciumphosphat der Formel
Ca₈(HPO₄)₂₊ₓ(PO₄)₄₋ₓ(OH)ₓ
ist, wobei 0 < x < 1.

11. Implantierbarer Stoff nach Anspruch 10, bei dem (a) das Phosphat apatitartiges Octacalciumphosphat ist, bei dem der Parameter x annähernd 0,5 (innerhalb von etwa ± 20%) ist, und die Formel annähernd
Ca₈(HPO₄)_{2,5}(PO₄)_{3,5}(OH)_{0,5}
ist.

12. Implantierbarer Stoff nach einem der Ansprüche 6 bis 11, bei dem (b) das Polyosid Dextran ist.

13. Implantierbarer Stoff nach Anspruch 12, bei dem der Gewichtsanteil des Dextrans im Verhältnis zu dem Gemisch zwischen 1% und 30% beträgt.

14. Implantierbarer Stoff nach Anspruch 8, bei dem (c) der Wirkstoff in der Form eines wasserlöslichen Salzes vorhanden ist.

15. Implantierbarer Stoff nach Anspruch 14, bei dem der Wirkstoff Netilmycin und/oder Gentamycin in der Form eines Sulfates ist.

16. Implantierbarer Stoff nach Anspruch 8 umfassend:
. einen Gewichtsanteil von Dextran, der im Verhältnis zu dem Gemisch zwischen 1% und 30% beträgt;
. einen Gewichtsanteil des Wirkstoffs, der im Verhältnis zu dem Gemisch zwischen 0,1% und 30% beträgt.
